# EUROPEAN PATENT SPECIFICATION

(11) **EP 3 266 767 B1**
(45) Date of publication and mention of the grant of the patent: **25.08.2021**
(21) Application number: 17180035.2
(22) Date of filing: 06.07.2017
(51) Int. Cl.: C07D 213/64, A61K 31/4412, A61P 9/00

(54) **METHOD FOR PREPARING AN ANTIFIBROTIC AGENT**
VERFAHREN ZUR HERSTELLUNG EINES ANTIFIBROTISCHEN MITTELS
PROCÉDÉ DE PRÉPARATION D'UN AGENT ANTIFIBROTIQUE

(30) Priority: 08.07.2016 IT 201600071672; 27.10.2016 IT 201600108927
(43) Date of publication of application: 10.01.2018
(73) Proprietor: Dipharma Francis S.r.l., 20021 Baranzate (MI) (IT)
(72) Inventor: ATTOLINO, Emanuele, 20021 BARANZATE (MI) (IT); MITTINO, Mauro, 20021 BARANZATE (MI) (IT); RAZZETTI, Gabriele, 20021 BARANZATE (MI) (IT); LOMBARDO, Alessandro, 20021 BARANZATE (MI) (IT); RESTELLI, Alessandro, 20021 BARANZATE (MI) (IT)
(74) Representative: Bianchetti & Minoja SRL

(56) References cited:
- WO-A1-03/014087
- WO-A1-2016/122420
- WO-A2-2010/141600
- WEI LONG ET AL: "A Copper-Based Metal-Organic Framework as an Efficient and Reusable Heterogeneous Catalyst for Ullmann and Goldberg Type C-N Coupling Reactions", MOLECULES ONLINE, vol. 20, no. 12, 27 December 2015 (2015-12-27), pages 21178-21192, XP055358988, DE ISSN: 1433-1373, DOI: 10.3390/molecules201219756

## Description

### FIELD OF THE INVENTION

The present invention relates to a new process for the synthesis of pirfenidone, an immunosuppressive drug, which has been studied in the clinics as a broad-spectrum anti-fibrotic agent and developed for example for the treatment of idiopathic pulmonary fibrosis.

### PRIOR ART

5-Methyl-1-phenylpyridin-2(1H)-one, known as pirfenidone, with the following formula **(I)** 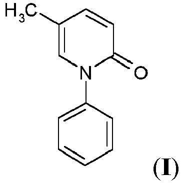 is a potent inhibitor of fibrogenic cytokines and TNF-α. Pirfenidone is well known as inhibitor of excessive biosynthesis and/or release of various fibrogenic cytokines such as TGF-β1, bFGF, PDGF and EGF. It has also been reported that pirfenidone is able to block the release of an excess of TNF-α from macrophages and other cells. Currently, pirfenidone is used in therapy, for example in the treatment of idiopathic pulmonary fibrosis.

The need of pirfenidone with a purity suitable to meet the regulatory requirements has stimulated the search for various alternative methods for its preparation, which at the same time had to be efficient and cost-effective from an industrial point of view.

For example, US 3,839,346 describes its preparation by a coupling reaction between a compound of formula (**II**) and an halobenzene of formula (**A**) 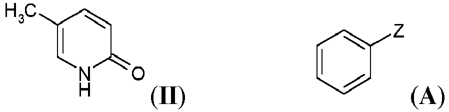 wherein **Z** is chlorine, bromine or iodine, and wherein the reaction is carried out in the presence of finely powdered metallic copper and an alkaline metal carbonate, for instance potassium carbonate. Preferably, the reaction is carried out in the absence of solvents and at a temperature between the melting point and the boiling point of the reagents.

US 8,519,140 describes the same "coupling" reaction between a compound of formula (**II**) as defined above and a compound of formula (**A**) as defined above, wherein **Z** is bromine, in the presence of Cu₂O and an inorganic base, for example, an alkaline metal hydroxide, an alkaline metal carbonate salt or a bicarbonate salt of an alkaline metal, in an organic solvent, such as dimethylformamide.

WO 2003/014087 describes a further method for preparing pirfenidone, wherein a compound of formula (**II**) as defined above is reacted with a compound of formula (**A**) as defined above, wherein **Z** is bromine or chlorine, in the presence of a copper (**I**) or copper (**II**) catalyst, preferably copper oxide, and a base.

Chinese patent application CN 101 891 676 discloses a further process for preparing pirfenidone, wherein a compound of formula (**II**) as defined above is reacted with a compound of formula **(A)** as defined above, wherein **Z** is bromine, in the presence of a copper **(I)** bromide, and potassium carbonate. Chinese patent application CN 1 817 862 discloses a process with cuprous chloride as catalyst and wherein a compound of formula (**II**) is iodobenzene. Both Chinese applications have in common that no further solvent is present and that the reaction is carried out at reflux temperature. Bromobenzene, as used in CN 101 891 676, has a boiling temperature of 156°C and iodobenzene, as used in CN 1 817 862, of 188°C.

Finally, WO 2016/122420, published on August 4, 2016, describes the preparation of pirfenidone by reaction of a compound of formula (**II**) with an halobenzene of formula **(A)** as defined above and wherein **Z** is bromine, in a polar aprotic solvent, in the presence of a copper-based **(I)** or copper (**II**) catalyst and a base, and wherein the aprotic polar solvent, in particular dimethylsulfoxide (DMSO), is used from 0.1 to 1 equivalents in volume compared to compound of formula (**II**). The inventors of the present invention have found that the reaction under such conditions would not be safe and could assume under adiabatic conditions an explosive character, since the freed reaction energy exceeds the one that can be absorbed by the reaction mixture.

The inventors of the present invention have found a new and safe alternative method for the preparation of pirfenidone, which thanks to the high yields is particularly suitable for an industrial production.

The new process provides a highly pure product, so as to meet regulatory requirements required for APIs, thanks to the particular reaction conditions and the choice of the ratios between the reagents, and also due to the fact that the *N*-arylation reaction is carried out in a suitable polar protic solvent or in the absence of reaction solvents.

### BRIEF DESCRIPTION OF THE FIGURES AND THE ANALYTICAL METHODS

Pirfenidone in crystalline form, herein referred to as Form I, was characterized by X-rays powder diffraction (XRPD). The X-ray diffraction spectra (XRPD) were collected with the Bruker D8 Advance diffractometer. The used detector is a PSD LynxEye detector. The used radiation is Cu Kd (λ = 1.541875 Å) filtered with nickel. X-ray diffraction spectra (XRPD) were collected in the range 2θ from 3° to 40° and with a step size of 0.02°.

The DSC pattern was obtained using a Mettler-Toledo DSC 822e differential scanning calorimeter with the following operating conditions: aluminum capsules, 30-250°C range at a scanning speed of 10°C/min, and nitrogen as purge gas (80 mL/min).

The particle size was determined using the laser light scattering technique using a Malvern Mastersizer 3000 instrument. 200 mg of the sample were dispersed in 2 mL of lecithin and immediately transferred into the measuring cell.

Figure 1 shows the XRPD spectrum of pirfenidone in crystalline form, herein defined as Form **I,** where the main peaks (expressed in ° in 2θ) are: 9.04; 14.53; 15.24; 18.62; 19.01; 20.13; 21.26; 22.26; 23.12; 24.58; 26.71; 27.03; 27.52; 30.55; and 32.59 ± 0.2° in 2θ.

Figure 2 shows the DSC pattern of crystalline pirfenidone, herein referred to as Form **I,** showing a peak at about 109°C.

### SUMMARY OF THE INVENTION

The object of the invention is a process for the preparation of pirfenidone, wherein an appropriate choice of the reactants of formula (**II**) and **(III),** as described below, of the catalyst, of the base, of their ratio and of the reaction conditions in a suitable polar protic solvent, or alternatively in the absence of solvents, surprisingly provides pirfenidone in a safe manner for the operators as well as with a high yield and purity.

The present disclosure also relates to pirfenidone in crystalline form, herein defined Form **I,** a method for its preparation, and a pharmaceutical composition containing said Form **I** as the active ingredient and one or more pharmaceutically acceptable excipients and/or carriers.

### DETAILED DESCRIPTION OF THE INVENTION

A first object of the invention is a process for preparing 5-methyl-1-phenyl-2(1H)-pyridone, also known as pirfenidone, having the following formula (I), 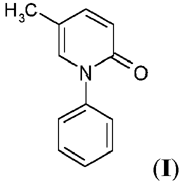 comprising the *N*-arylation reaction of a pyridone of formula (**II**) with a compound of formula **(III),** wherein X is a halogen atom, 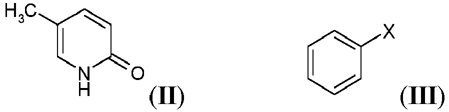 wherein the reaction is carried out in the presence of a copper salt of formula CuY, wherein Y is a halogen, in the presence of a base and of a polar protic solvent.

Alternatively, the process for preparing pirfenidone of formula **(I)** as defined above may be carried out in the absence of solvents.

The halogen substituent X in a compound of formula **(III)** may be a chloride, bromide or iodide atom, preferably bromide.

The halogen substituent Y in a copper salt of formula CuY may be a bromide or iodide atom, preferably iodide.

A base may be a carbonate salt or a bicarbonate salt of an alkaline metal, typically lithium, potassium or sodium carbonate or bicarbonate, preferably potassium carbonate. Said base may be typically used in an at least stoichiometric quantity in respect to the pyridone of formula (**II**).

The polar protic solvent is an alcohol selected from 1-butanol, 2-butanol, *tert-*butanol, 1-pentanol, 2-pentanol, 3-pentanol, 1-cyclohexanol, 1-heptanol, ethylene glycol, glycerin, preferably 1-butanol, 2-butanol, *tert*-butanol, cyclohexanol, more preferably 1-butanol.

The *N*-arylation reaction, both in the presence of a polar protic solvent and in the absence of solvents, may be advantageously carried out using about 1.6 to about 1.2 moles of a compound of formula **(III),** particularly bromobenzene, per mole of pyridone of formula (**II**), preferably from about 1.5 to about 1.3 moles, more preferably about 1.3 moles.

A copper salt of the formula CuY, wherein the halogen substituent Y is as defined above and is preferably iodine, in the absence or in the presence of a solvent, may be used in an amount of about 5 to 20% by weight with respect to the amount of pyridone of formula (**II**), preferably between about 8 and 18%, more preferably between about 14 and 18%. It has been surprisingly found that said amount of copper salt allows to fully convert the pyridone of formula (**II**). In addition, the salt may be completely removed at the end of reaction.

The reaction in the presence of a polar protic solvent may be performed at temperatures above room temperature. The reaction may be carried out, for example, at a temperature of at least about 50°C, or at least about 70°C, preferably at least about 90°C, at least about 100°C, or at least about 110°C.

In the presence of a polar protic solvent, for example 1-butanol, the reaction may be preferably carried out at a temperature between about 120°C and about 140°C, more preferably about 120-128°C.

In the absence of a solvent, the *N*-arylation reaction may be carried out at a temperature ranging from 100°C to 140°C, preferably at a temperature ranging from 120°C to 140°C, more preferably from about 130°C to about 138°C.

In the absence of solvent, the reaction time is about 14 to 17 hours, typically about 16 hours, whereas in the presence of a polar protic solvent the reaction is carried out within about 16 to 48 hours, typically within about 24 hours.

As indicated above, the *N*-arylation reaction may be carried out in the absence of solvents. In fact, the inventors of the present invention have found that the compound of formula **(III),** particularly bromobenzene, when used in the above-defined amount, acts as arylating agent and reaction solvent.

The *N*-arylation reaction performed in the absence of solvents, overcomes the technical problem associated to the presence of residual solvent traces, as is the case, for example, of the commonly used dimethylformamide, which -if present in the final product as impurity- results to be difficult and laborious to be eliminated. In addition, according to the ICH (Q3C) guidelines DMF (for example as used in US 8,519,140) is a class 2 solvent with a limit of 880 ppm. Thus, it is advisable to avoid said solvent.

The same technical problem directed to the presence of residual solvent traces, is surprisingly solved by the use of a protic polar solvent as defined herein. The inventors of the present invention have found that the recrystallization of pirfenidone of formula **(I),** prepared in a polar protic solvent, for instance 1-butanol, from isopropanol provides a product, wherein the content of the solvents (in particular 1-butanol or isopropanol) is well below the limits of the ICH (Q3C) guidelines for residual solvents. For example, in case the reaction is carried out in 1-butanol, pirfenidone of formula **(I)** once crystallized from isopropanol has a content of 1-butanol well below 50 ppm, thus over 100 times lower than the limit of 5000 ppm specified for a class 3 solvent such as 1-butanol. If desired, a further recrystallization from isopropanol allows reducing further the solvent content.

In addition, the use of a protic polar solvent, contrary to the case with the use of DMSO as reported in WO 2016/122420, results in essentially safer reaction conditions, since even under adiabatic conditions the reaction mixture may not develop such exothermic energy to give origin to explosive developments. The operators can perform thus the reaction under non-hazardous operating conditions without additional precautions. Thus, the process becomes more easily applicable at an industrial level.

At the herein disclosed conditions, the *N*-arylation reaction according to the present invention proceeds with a conversion greater than 99%.

Thus, thanks to the particular reaction conditions and reagents, a compound of formula (I) obtained by this process has already a chemical purity determined by HPLC at 220 nm greater than 94% (Area %), typically around 95%.

The reaction mixture containing the product pirfenidone may be purified by known methods. For example, pirfenidone may be extracted from the reaction mixture with toluene. The organic phase comprising pirfenidone may be then treated with ammonia at about 10% to remove the copper salt catalyst, and then with an aqueous saline solution of about 5% to about 25%, preferably from about 10 to about 20%, of sodium chloride. The residual organic phase may be then concentrated at reduced pressure, preferably under vacuum, at an internal temperature comprised between 60°C and 80°C to dryness, in order to obtain pirfenidone as crystalline solid.

At this point, the product may be recrystallized to further increase the degree of purity.

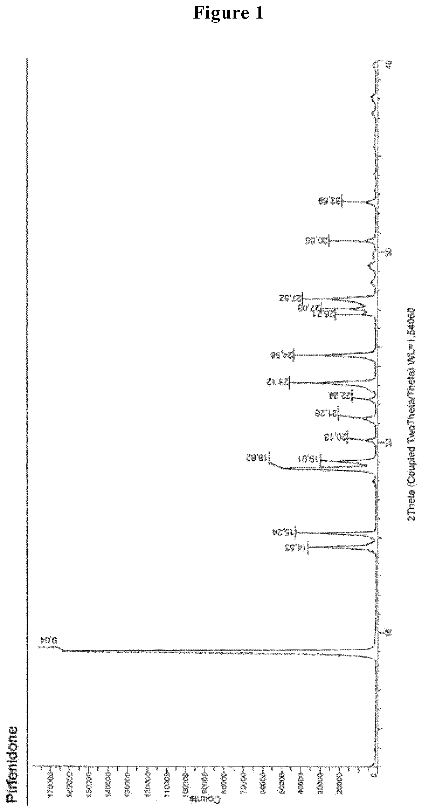

According to a further embodiment, the present invention is directed to pirfenidone in crystalline form, herein defined as Form **I,** having a XRPD spectrum as illustrated in Figure 1, wherein the most intensive diffraction peaks are at: 9.04; 14.53; 15.24; 18.62; 19.01; 20.13; 21.26; 22.26; 23.12; 24.58; 26.71; 27.03; 27.52; 30.55; and 32.59 ± 0.2° in 2θ.

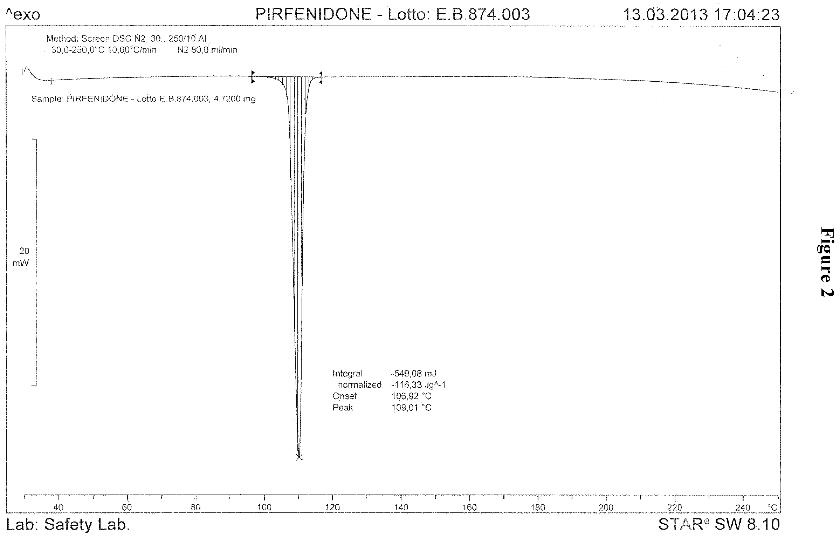

The same crystalline Form **I** has a DSC pattern as shown in Figure 2, showing a peak at about 109°C.

The present invention also provides a method for obtaining pirfenidone in crystalline Form **I,** as defined above, by a process comprising:
- dissolution of previously obtained pirfenidone in isopropanol;
- cooling of the solution down to a temperature equal or below 10°C;
- keeping the solution at said temperature;
- optionally seeding with a seed of crystalline Form I previously obtained; and
- recovering of the crystalline solid.

The dissolution of pirfenidone in isopropanol may be performed by heating the dispersion of pirfenidone in isopropanol up to the boiling point of the solvent until complete dissolution, preferably 50 to 75°C.

The cooling of the solution containing pirfenidone may be carried out in a time varying from about 3 hours to about 5 hours, preferably in about 4 hours, bringing the temperature to about 10°C or below, preferably to about 0°C or below, more preferably between about -5°C and about -15°C.

The cooled solution may be maintained at said temperature for a time ranging from about 0.2 to about 5 hours, preferably ranging from about 1 to about 3 hours.

The crystallization may be promoted seeding it with a seed of previously obtained crystalline pirfenidone in crystalline Form **I.**

The crystalline solid may be recovered according to known methods, for example by filtration or centrifugation, preferably by filtration on a Büchner funnel. The product may then be dried under vacuum at a temperature between about 45°C and 60°C for about 10 to about 15 hours providing pirfenidone in crystalline Form **I** with yields typically exceeding 66%.

Pirfenidone in crystalline Form **I** obtained by the above method has a chemical purity, evaluated by HPLC at 220 nm, equal to or greater than 99.8% (Area %), preferably equal to or greater than 99.9%, more preferably equal to or greater than 99.97%, wherein each impurity is present in a percentage equal to or lower than 0.05%, preferably equal to or lower than 0.01%, and wherein a compound of formula **(II)** as an impurity is typically present in a percentage equal to or lower than 0.01%, preferably equal to or less than 0.006%. Thus, a compound of formula (**II**) is substantially absent, and well lower than 0.05% required by regulatory authorities.

The dimension of the crystals of pirfenidone crystalline Form **I,** obtainable according to the procedure disclosed above, is characterized by a D₅₀ value comprised between about 25 and 250 µm, preferably between about 100 and 150 µm. If desired, the size may be reduced by micronisation or end milling.

A further aspect of the present disclosure is a pharmaceutical composition comprising pirfenidone in crystalline Form **I,** in particular having the chemical purity as disclosed above, as active pharmaceutical ingredient, and one or more pharmaceutically acceptable excipients and/or carriers.

The same composition may contain one or more further active pharmaceutical ingredients, typically from 1 to 3, typically antibiotics, such as for example ciprofloxacin. The pharmaceutical composition, preferably in solid form, may be prepared according to known methods.

The dosages of pirfenidone in crystalline Form **I,** and the optional further active pharmaceutical ingredients present in the composition, may be those already commonly known in therapy.

A further aspect of the present disclosure is pirfenidone in crystalline Form I for the use as medicament, either alone or in combination with one or more further active pharmaceutical ingredients, typically from 1 to 3, for example antibiotics, for example ciprofloxacin.

An additional aspect of the present disclosure is pirfenidone in crystalline Form I for the use as immunosuppressant medicament.

An additional aspect of the present disclosure is pirfenidone in crystalline Form I for the use in the treatment of idiopathic pulmonary fibrosis.

A compound of formula **(III)** is a known compound, for example from US 3,839,346, and may be prepared according to known methods.

The following examples further illustrate the invention.

### Example 1 - Synthesis of pirfenidone in crystalline Form I

5-Methyl-2-pyridone (200 g), potassium carbonate (305 g), bromobenzene (374 g) and copper iodide (35.2 g) are placed under nitrogen atmosphere in a 2 L glass reactor equipped with a mechanical stirrer and a reflux condenser. The mixture is heated to about 135-138°C for about 12 h. Once the conversion is greater than 99%, the mixture is cooled down to about 80°C, demineralized water (400 g) and toluene (432 g) are added and the biphasic mixture is cooled to a temperature of about 70°C. Then, the stirring is stopped and the biphasic mixture is filtered. The filtered mixture is placed into the 21 reactor and then the aqueous phase is separated. The organic phase is washed with a series of solutions of 10% ammonia (280 g) and sodium chloride (25 g). Subsequently, the organic phase is concentrated under vacuum at an internal temperature of about 70°C to dryness.

The residue is then repeatedly treated with isopropanol (314-236 g) to remove the present toluene. Further isopropanol (236 g) is then added and the reaction mixture is heated to complete dissolution and then cooled down to -10°C within about 4 hours. The mixture is kept under these conditions for at least 1 hour, then filtered on a Büchner funnel. The solid is washed then twice with isopropanol (94 g) providing approximately 254 g of a wet product, which dried in a vacuum oven at 55°C for about 12 hours provides 224 g of pirfenidone as a crystalline solid (yield 66%). The crystalline solid exhibits an XRPD spectrum as shown in Figure 1, wherein the most intense peaks are at 9.04; 14.53; 15.24; 18.62; 19.01; 20.13; 21.26; 22,26; 23.12; 24.58; 26.71; 27.03; 27.52; 30.55; and 32.59 ± 0.2° in 2θ. Said crystalline form also has a DSC pattern as shown in Figure 2, showing a peak at about 109°C.

### Example 2 - Synthesis of pirfenidone in crystalline Form I

5-Methyl-2-pyridone (250 g), potassium carbonate (380 g), bromobenzene (468 g), 250 mL of 1-butanol and copper (I) iodide (43.8 g) are placed under nitrogen atmosphere in a 2 L glass reactor equipped with a mechanical stirrer and reflux condenser. The mixture is heated to a temperature of about 122-128°C for about 24 hours, then cooled down to about 65-70°C. Toluene (625 mL), demineralized water (875 g) and NH₄OH 30% -33% (138 mL) are added, while maintaining the temperature at about 60-70°C. After about 15-20 minutes, the aqueous phase is discarded and the organic phase is washed 3 times with a solution of NaCl (12.5 g) in demineralized water (250 mL) and with NH₄OH 30% to 33% (28 mL). The organic phase is then concentrated under vacuum at an internal temperature of about 50-70°C to dryness.

The residue is then treated with isopropanol to remove all toluene still present, then dissolved in isopropanol (250 mL) at a temperature of about 65 to about 70°C. The obtained solution is cooled down to 50-55°C, heated again until complete dissolution and then cooled within about 4 hours down to -10°C. The mixture is kept at these conditions for at least 30 minutes, then the resulting solid is filtered and washed with isopropanol (125 mL), previously cooled down to about -5/-10°C. About 280 g of a wet product is obtained, which once dried in a vacuum oven at 50°C for about 12 to 20 hours provides 263 g of pirfenidone as crystalline solid. Yield 62%.

### Example 3 - Recrystallization of pirfenidone of crystalline Form I

Pirfenidone obtained according to Examples 1 or 2 (1960 g) and isopropanol (1540 g) are placed under nitrogen atmosphere into a 2 L glass reactor equipped with a mechanical stirrer and reflux condenser. It is not necessary that the product has been dried beforehand. The mixture is heated until complete dissolution and about 16 g of carbon are added. The hot solution is filtered on a perlite panel, cooled within about 4 hours down to -0°C and maintained at these conditions for at least one further hour. After filtration on a Büchner funnel, the solid is washed twice with isopropanol (157 g) providing approximately 1901 g of a wet product, which is dried in a vacuum oven at about 55°C for about 12 hours to give 1776 g of pirfenidone. (yield: 90.6%)

The mother liquors and washing solutions (about 2670 mL, about 2100 g and containing about 165 g of product) are concentrated to isolate further product.

Pirfenidone is obtained in crystalline Form **I** as defined herein, with a spectrum (XRPD) as shown in Figure 1, wherein the most intense diffraction peaks are at 9.04; 14.53; 15.24; 18.62; 19.01; 20.13; 21.26; 22,26; 23.12; 24.58; 26.71; 27.03; 27.52; 30.55; and 32.59 ± 0.2° in 2θ. Said crystalline form also has a DSC pattern as shown in Figure 2, showing a peak at about 109°C. The purity is about 99.97% (HPLC at 220 nm) and the compound of formula (**II**) as impurity is present in a percentage of 0.006%.

## Claims

1. A process for preparing 5-methyl-1-phenyl-2(1H)-pyridone (pirfenidone) of formula (I) 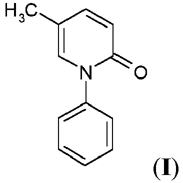 comprising the *N*-arylation reaction of a pyridone of formula (**II**) with a compound of formula (III), wherein X is a halogen atom, 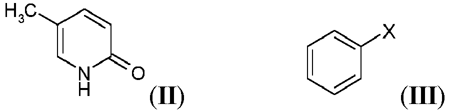 and wherein the reaction is carried out in presence of a copper salt of formula CuY, wherein Y is a halogen, of a base and of a polar protic solvent,
wherein the polar protic solvent is an alcohol selected from 1-butanol, 2-butanol, *tert-*butanol, 1-pentanol, 2-pentanol, 3-pentanol, 1-cyclohexanol, 1-heptanol, ethylene glycol, glycerin.

2. The process according to claim 1, wherein the halogen X in a compound of formula **(III)** is chloride, bromide or iodide, preferably bromide.

3. The process according to claims 1 or 2, wherein the halogen Y of the copper salt of formula CuY is bromide or iodide, preferably iodide.

4. The process according to claims 1 to 3, wherein the polar protic solvent is chosen from 1-butanol, 2-butanol, *tert*-butanol, and 1-cyclohexanol.

5. The process according to claims 1 to 4, wherein the polar protic solvent is 1-butanol.

6. The process according to claims 1 to 5, wherein the copper salt is used in amount ranging from about 5 to about 20 wt. %, preferably from about 8 to about 18 wt. %, more preferably from about 14 to about 18 wt. %, in respect to the amount of the pyridone of formula (**II**).

7. The process according to claims 1 to 6, wherein the base is an alkali metal carbonate or bicarbonate salt, typically a lithium carbonate or bicarbonate, potassium carbonate or bicarbonate, or sodium carbonate or bicarbonate, preferably potassium carbonate.

8. The process according to claims 1 to 7, wherein the *N*-arylation reaction is carried out using between about 1.6 moles and about 1.2 moles of a compound of formula **(III),** in particular bromobenzene, for one mole of pyridone of formula (**II**), preferably from about 1.5 moles to about 1.3 moles, more preferably about 1.3 moles.

9. The process according to claims 1 to 8, wherein the *N*-arylation reaction is carried out at a temperature ranging from about 120°C to about 140°C, preferably about 120-128°C.

10. The process according to claims 1 to 8, wherein the *N*-arylation reaction is carried out in the absence of solvent at a temperature ranging from 100°C to 140°C, preferably at a temperature ranging from 120°C to 140°C, more preferably from about 130°C to about 138°C.

11. A process for preparing pirfenidone of formula (I) according claims 1 to 10, further comprising:
- dissolution of pirfenidone of formula **(I)** in isopropanol;
- cooling of the solution at a temperature equal or below 10°C;
- maintaining the solution at such temperature; and
- recovering pirfenidone in crystalline Form **I** having an XRPD spectrum as shown in Figure 1, wherein the most intense peaks fall at 9.04; 14.53; 15.24; 18.62; 19.01; 20.13; 21.26; 22.26; 23.12; 24.58; 26.71; 27.03; 27.52; 30.55; and 32.59 ± 0.2° in 2θ.

12. A process for preparing pirfenidone of formula (I) according to claim 11, wherein the solution cooled down to a temperature equal or below 10°C is seeded with a previously obtained seed crystal of pirfenidone in crystalline Form **I.**

## Patentansprüche

1. Verfahren zur Herstellung von 5-Methyl-1-phenyl-2(1H)-pyridon (Pirfenidon) der Formel **(I)** 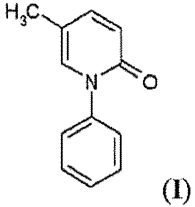 umfassend die N-Arylierungsreaktion eines Pyridons der Formel (**II**) mit einer Verbindung der Formel (**III**), wobei X ein Halogenatom ist, 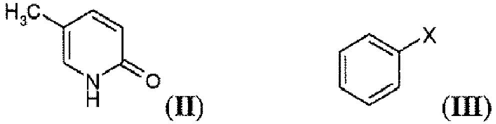 und wobei die Reaktion in Gegenwart eines Kupfersalzes der Formel CuY, wobei Y ein Halogen ist, einer Base und eines polaren protischen Lösungsmittels durchgeführt wird,
wobei das polare protische Lösungsmittel ein Alkohol ist, ausgewählt aus 1-Butanol, 2-Butanol, tert-Butanol, 1-Pentanol, 2-Pentanol, 3-Pentanol, 1-Cyclohexanol, 1-Heptanol, Ethylenglykol, Glycerin.

2. Verfahren nach Anspruch 1, wobei das Halogen X in einer Verbindung der Formel **(III)** Chlorid, Bromid oder Iodid, vorzugsweise Bromid, ist.

3. Verfahren nach Anspruch 1 oder 2, wobei das Halogen Y des Kupfersalzes der Formel CuY Bromid oder Iodid, vorzugsweise Iodid, ist.

4. Verfahren nach einem der Ansprüche 1 bis 3, wobei das polare protische Lösungsmittel aus 1-Butanol, 2-Butanol, *tert-*Butanol und 1-Cyclohexanol ausgewählt ist.

5. Verfahren nach einem der Ansprüche 1 bis 4, wobei das polare protische Lösungsmittel 1-Butanol ist.

6. Verfahren nach einem der Ansprüche 1 bis 5, wobei das Kupfersalz in einer Menge im Bereich von etwa 5 bis etwa 20 Gew.-%, vorzugsweise von etwa 8 bis etwa 18 Gew.-%, bevorzugter von etwa 14 bis etwa 18 Gew.-%, bezogen auf die Menge des Pyridons der Formel (**II**), verwendet wird.

7. Verfahren nach einem der Ansprüche 1 bis 6, wobei die Base ein Alkalimetallcarbonat- oder -bicarbonatsalz, typischerweise ein Lithiumcarbonat oder -bicarbonat, Kaliumcarbonat oder -bicarbonat oder Natriumcarbonat oder -bicarbonat, vorzugsweise Kaliumcarbonat, ist.

8. Verfahren nach einem der Ansprüche 1 bis 7, wobei die N-Arylierungsreaktion unter Verwendung von etwa 1,6 Mol bis etwa 1,2 Mol einer Verbindung der Formel (**III**), insbesondere Brombenzol, für ein Mol Pyridon der Formel (**II**) durchgeführt wird, vorzugsweise von etwa 1,5 Mol bis etwa 1,3 Mol, bevorzugter etwa 1,3 Mol.

9. Verfahren nach einem der Ansprüche 1 bis 8, wobei die N-Arylierungsreaktion bei einer Temperatur im Bereich von etwa 120°C bis etwa 140°C, vorzugsweise etwa 120-128°C, durchgeführt wird.

10. Verfahren nach einem der Ansprüche 1 bis 8, wobei die N-Arylierungsreaktion in Abwesenheit von Lösungsmittel bei einer Temperatur im Bereich von 100°C bis 140°C, vorzugsweise bei einer Temperatur im Bereich von 120°C bis 140°C, bevorzugter von etwa 130°C bis etwa 138°C, durchgeführt wird.

11. Verfahren zur Herstellung von Pirfenidon der Formel **(I)** nach den Ansprüchen 1 bis 10, ferner umfassend:
- Auflösen von Pirfenidon der Formel **(I)** in Isopropanol;
- Abkühlen der Lösung auf eine Temperatur gleich oder unter 10°C
- Halten der Lösung bei einer solchen Temperatur; und
- Gewinnen von Pirfenidon in kristalliner Form I mit einem XRPD-Spektrum wie in Figur 1 gezeigt, wobei die intensivsten Peaks bei 9,04; 14,53; 15,24; 18,62; 19,01; 20,13; 21,26; 22,26; 23,12; 24,58; 26,71; 27,03; 27,52; 30,55 und 32,59 ±0,2° in 2θ liegen.

12. Verfahren zur Herstellung von Pirfenidon der Formel **(I)** nach Anspruch 11, wobei die auf eine Temperatur gleich oder unter 10°C abgekühlte Lösung mit einem vorher erhaltenen Impfkristall von Pirfenidon in kristalliner Form **I** geimpft wird.

## Revendications

1. Procédé destiné à la préparation de la 5-méthyl-1-phényl-2(1H)-pyridone (pirfénidone) qui répond à la formule **(I)** : 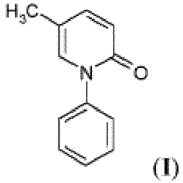 qui comprend la réaction de *N*-arylation d'une pyridone répondant à la formule (**II**) avec un composé répondant à la formule (**III**) dans laquelle X représente un atome d'halogène : 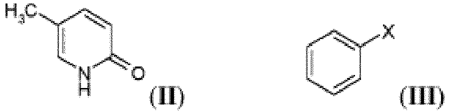 et dans lequel la réaction est mise en œuvre en présence d'un sel de cuivre répondant à la formule CuY, dans laquelle Y représente un atome d'halogène, d'une base et d'un solvant protique polaire ;
dans lequel le solvant protique polaire représente un alcool qui est choisi parmi le 1-butanol, le 2-butanol, le tert-butanol, le 1-pentanol, le 2-pentanol, le 3-pentanol, le 1-cyclohexanol, le 1-heptanol, l'éthylène glycol, le glycérol.

2. Procédé selon la revendication 1, dans lequel l'atome d'halogène X dans un composé répondant à la formule (**III**) représente un chlorure, un bromure ou un iodure, de préférence un bromure.

3. Procédé selon la revendication 1 ou 2, dans lequel l'atome d'halogène Y du sel de cuivre répondant à la formule CuY représente un bromure ou un iodure, de préférence un iodure.

4. Procédé selon les revendications 1 à 3, dans lequel le solvant protique polaire est choisi parmi le 1-butanol, le 2-butanol, le tert-butanol et le 1-cyclohexanol.

5. Procédé selon les revendications 1 à 4, dans lequel le solvant protique polaire est le 1-butanol.

6. Procédé selon les revendications 1 à 5, dans lequel le sel de cuivre est utilisé en une quantité qui se situe dans la plage d'environ 5 à environ 20 % en poids, de préférence d'environ 8 à environ 18 % en poids, de manière plus préférée d'environ 14 à environ 18 % en poids, par rapport à la quantité de la pyridone répondant à la formule (**II**).

7. Procédé selon les revendications 1 à 6, dans lequel la base représente un sel carbonate ou bicarbonate d'un métal alcalin, de manière spécifique un carbonate ou un bicarbonate de lithium, un carbonate ou un bicarbonate de potassium ou un carbonate ou un bicarbonate de sodium, de préférence le carbonate de potassium.

8. Procédé selon les revendications 1 à 7, dans lequel la réaction de N-arylation est mise en œuvre en utilisant entre environ 1,6 mol et environ 1,2 mol d'un composé répondant à la formule (**III**), en particulier du bromobenzène, pour une mole de pyridone répondant à la formule (**II**), de préférence pour environ 1,5 mol à environ 1,3 mol, de manière plus préférée pour environ 1,3 mol.

9. Procédé selon les revendications 1 à 8, dans lequel la réaction de N-arylation est mise en œuvre à une température qui se situe dans la plage d'environ 120 °C à environ 140 °C, de préférence d'environ 120 à 128 °C.

10. Procédé selon les revendications 1 à 8, dans lequel la réaction de N-arylation est mise en œuvre en l'absence de solvant à une température qui se situe dans la plage de 100 °C à 140 °C, de préférence à une température qui se situe dans la plage de 120 °C à 140 °C, de manière plus préférée dans la plage d'environ 130 °C à 138 °C.

11. Procédé destiné à la préparation de la pirfénidone répondant à la formule **(I)** selon les revendications 1 à 10, qui comprend en outre :
- la dissolution de la pirfénidone répondant à la formule **(I)** dans de l'isopropanol ;
- le refroidissement de la solution à une température qui est égale ou qui est inférieure à 10 °C ;
- le maintien de la solution à ladite température ; et
- la récupération de la pirfénidone dans la forme cristalline **I** dont le spectre XRPD est tel que représenté en figure 1, dans laquelle les pics les plus intenses se retrouvent à 9,04 ; 14,53 ; 15,24 ; 18,62 ; 19,01 ; 20,13 ; 21,26 ; 22,26 ; 23,12 ; 24,58 ; 26,71 ; 27,03 ; 27,52 ; 30,55 ; et 32,59 ± 0,2° dans l'angle 20.

12. Procédé destiné à la préparation de la pirfénidone répondant à la formule **(I)** selon la revendication 11, dans laquelle on ensemence la solution qui a été refroidie jusqu'à une température égale ou inférieure à 10 °C avec un germe cristallin, que l'on a obtenu précédemment, de pirfénidone dans la forme cristalline **I.**
